# EUROPEAN PATENT APPLICATION

(11) **EP 3 388 047 A1**
(43) Date of publication of application: **17.10.2018**
(21) Application number: 18165866.7
(22) Date of filing: 05.04.2018
(51) Int. Cl.: A61K 8/11, A61K 8/92, A61K 8/36, A61Q 5/12

(54) **MICROCAPSULE AND METHOD FOR PRODUCING MICROCAPSULE, AS WELL AS COSMETIC COMPOSITION AND METHOD FOR PRODUCING COSMETIC COMPOSITION**

(30) Priority: 10.04.2017 JP 2017077599
(71) Applicant: Panasonic Intellectual Property Management Co., Ltd., Osaka-shi, Osaka 540-6207 (JP)
(72) Inventor: ISHIHARA, Aya, Osaka-shi, Osaka 540-6207 (JP); INOUE, Hiroyuki, Osaka-shi, Osaka 540-6207 (JP); HANATO, Yumi, Osaka-shi, Osaka 540-6207 (JP); TANAKA, Masato, Niigata City, Niigata 950-2181 (JP); TAGUCHI, Yoshinari, Niigata City, Niigata 950-2181 (JP); SAITO, Natsukaze, Niigata City, Niigata 950-2181 (JP)
(74) Representative: Grünecker Patent- und Rechtsanwälte PartG mbB

(57) **Abstract**

A microcapsule according to the present disclosure is composed of a core substance and a wall substance including the core substance in the wall substance. The core substance is an anionic hydrophobic substance. The wall substance is a cationic polymer. The microcapsule is smaller than 200 µm in particle size. Thus, the microcapsule is provided which is likely to give attachment to hair and unlikely to damage the hair.

## Description

### BACKGROUND

### 1. Technical Field

The present disclosure relates to a microcapsule, a cosmetic composition containing the microcapsule, and methods for producing the microcapsule and the cosmetic composition.

### 2. Description of the Related Art

A microcapsule has a coating layer as a wall film substance formed around a functional included substance to serve as a core substance for protecting the functional included substance. In recent years, microcapsules are used in various fields, depending on the combination of a core substance to be included and a wall film substance coating the core substance. The fields include, for example, cosmetic compositions and the like containing microcapsules.

Microcapsules themselves are extremely small microparticles, and the shells (hereinafter, also referred to as "shells") of the microcapsules are also extremely thin. Therefore, the microcapsules have a problem of dissolving the core component (core substance) in the shells, or a problem of eluting the core component (core substance) into an external environment due to diffusion and osmosis through microscopic pores in the shells. Therefore, development of microcapsules with sufficiently dense or strong shells has been desired.

For example, Unexamined Japanese Patent Publication No. 2010-253375 discloses a method for producing a microcapsule, where a hydrophobic core substance is dispersed in an acid solution of a polysaccharide polymer to prepare an oil-in-water emulsion solution (hereinafter, may be referred to as an o/w solution (Oil-in-water solution)), the o/w solution and an anionic polymer are mixed to undergo phase separation (coacervation process), and an alkali metal salt of a carboxylic acid is then added to cause a wall substance to cure, and a microcapsule obtained by the production method. The microcapsulation method described in Unexamined Japanese Patent Publication No. 2010-253375 makes it easy to adjust a capsule film thickness, thereby making it easy to control a sustained-release property of the core substance in the microcapsule to be obtained.

### SUMMARY

However, the microcapsulation method described in Unexamined Japanese Patent Publication No. 2010-253375 has a problem of difficulty with attachment to negatively charged hair, because the outermost layer of microcapsule is an anionic polymer. In addition, the microcapsulation method mentioned above also has a problem of possibly damaging hair with increased pH of the microcapsule dispersion, because of use of an alkali metal salt.

Therefore, the present disclosure has been made in view of the problems mentioned above, and an object of the disclosure is to provide a microcapsule which is likely to give attachment to hair and unlikely to damage the hair and a method for producing the microcapsule, as well as a cosmetic composition and a method for producing the cosmetic composition.

In order to achieve the object mentioned above, a microcapsule according to an exemplary embodiment of the present disclosure is a microcapsule composed of a core substance and a wall substance encapsulating the core substance, in which the core substance is an anionic hydrophobic substance, the wall substance is a cationic polymer, and the microcapsule is smaller than 200 µm in particle size.

In addition, in order to achieve the object mentioned above, a cosmetic composition according to an exemplary embodiment of the present disclosure includes a plurality of the microcapsules mentioned above and a water phase that disperses the plurality of microcapsules, in which the plurality of microcapsules have an average particle size of 3 µm ± 1 µm and a standard deviation of 0.3 ± 0.1.

Furthermore, in order to achieve the object mentioned above, a method for producing a microcapsule according to an exemplary embodiment of the present disclosure is a method for producing a microcapsule composed of a core substance and a wall substance including the core substance in the wall substance, the method including: an oil-in-water emulsion solution preparation step of preparing an oil-in-water emulsion solution of the core substance which is an anionic hydrophobic substance by dispersing, in a water phase, the core substance; and an inclusion step of including, in the wall substance which is a cationic polymer, microparticles of the core substance in the oil-in-water emulsion solution by mixing the oil-in-water emulsion solution with the wall substance, and the microcapsule is smaller than 200 µm in particle size.

In addition, in order to achieve the object mentioned above, a method for producing a cosmetic composition according to an exemplary embodiment of the present disclosure includes the above-mentioned method for producing a microcapsule.

The present disclosure provides a microcapsule which is likely to give attachment to hair and unlikely to damage the hair and a method for producing the microcapsule, as well as a cosmetic composition and a method for producing the cosmetic composition.

### BRIEF DESCRIPTION OF THE DRAWINGS

FIG. 1A is a schematic sectional view illustrating an example of a microcapsule according to a first aspect of an exemplary embodiment;
FIG. 1B is a process chart showing an example of a method for producing the microcapsule and a cosmetic composition according to the first aspect of the exemplary embodiment;
FIG. 1C is a graph showing results of measuring particle size distribution of the microcapsule according to the first aspect of the exemplary embodiment;
FIG. 2A is a schematic sectional view illustrating an example of a microcapsule according to a second aspect of an exemplary embodiment;
FIG. 2B is a process chart showing an example of a method for producing the microcapsule and a cosmetic composition according to the second aspect of the exemplary embodiment;
FIG. 2C is a process chart showing another example of the method for producing the microcapsule and the cosmetic composition according to the second aspect of the exemplary embodiment;
FIG. 3A is a schematic sectional view illustrating an example of a microcapsule according to a third aspect of an exemplary embodiment;
FIG. 3B is a process chart showing an example of a method for producing the microcapsule and a cosmetic composition according to the third aspect of the exemplary embodiment;
FIG. 4A is a schematic sectional view illustrating an example of a microcapsule according to a fourth aspect of an exemplary embodiment;
FIG. 4B is a process chart showing an example of a method for producing the microcapsule and a cosmetic composition according to the fourth aspect of the exemplary embodiment;
FIG. 5 is a schematic configuration diagram illustrating an example of a hair-care device that includes a cosmetic composition according to the present disclosure; and
FIGS. 6A to C-2 are diagrams illustrating atomization of cosmetic compositions according to examples.

### DETAILED DESCRIPTION

Hereinafter, an exemplary embodiment of the present disclosure will be described with reference to the drawings. It is to be noted that the exemplary embodiments to be described below are all intended to give a preferred specific example of the present disclosure. Therefore, numeric values, shapes, materials, constituents, arrangement positions and connection modes of the constituents, steps and orders of the steps, and the like, which are presented in the following exemplary embodiment, are merely example, and not intended to limit the present disclosure. Accordingly, among the constituents in the following exemplary embodiment, constituents which are not recited in the independent claims for the most generic concept are described as arbitrary constituents.

It is to be noted that the respective drawings are considered pattern diagrams which are not necessarily shown in a rigorous manner. In addition, in the respective drawings, substantially the same configurations are denoted by the same reference numerals, and repeated description will be omitted or simplified.

### (Exemplary embodiment)

A microcapsule and a method for producing the microcapsule, as well as a cosmetic composition and a method for producing the cosmetic composition according to the present exemplary embodiment will be described below with reference to the drawings.

### [A. Microcapsule and cosmetic composition]

First, features each common to the microcapsule and the cosmetic composition according to the present exemplary embodiment will be described. It is to be noted that production methods will be described in respective aspects.

Microcapsule 10 according to the present exemplary embodiment is composed of core substance 1 and wall substance 2 including therein core substance 1. Core substance 1 is an anionic hydrophobic substance, whereas wall substance 2 is a cationic polysaccharide polymer. Therefore, the microcapsule can be electrostatically formed by a difference in surface charge between anionic core substance 1 and cationic wall substance 2. In addition, a thickness of wall substance 2 of the microcapsule can be adjusted depending on anionic strength of core substance 1. Thus, microcapsule 10 can be adjusted to desired strength.

In addition, microcapsule 10 according to the present exemplary embodiment is smaller than 200 µm in particle size. Since mist discharge outlet port 113a of hair-care device 100 to be described later is 200 µm in hole diameter, it is possible to make a cosmetic composition containing microcapsule 10 into a form of a mist (electrostatic atomization) in an appropriate manner with use of hair-care device 100, as long as microcapsule 10 is smaller than 200 µm in particle size. In addition, even when hair is touched with a hand after microcapsule 10 is attached to the hair, there is no feeling of strangeness such as stickiness, but good usability such as affinity to hair is obtained.

Core substance 1 according to the present exemplary embodiment may have a form of a liquid. Core substance 1 is a hydrophobic substance in the form of a liquid at ordinary temperature, thereby making core substance 1 likely to be released from inside of microcapsule 10 when microcapsule 10 is broken. In addition, core substance 1 in the form of a liquid is thus easily applied to hair.

Core substance 1 has only to be any anionic hydrophobic substance, and examples of the material for core substance 1 may include oils and fats such as avocado oil, olive oil, cacao fat, beef fat, wheat germ oil, camellia oleifera seed oil, safflower oil, soybean oil, tea seed oil, evening primrose oil, camellia oil, tea tree oil, palm kernel oil, palm oil, castor oil, sunflower oil, macadamia nut oil, manuka oil, horse fat, cottonseed oil, Japan wax, moringa oil, and coconut oil; silicones such as methylpolysiloxane, methylphenylpolysiloxane, cyclic dimethyl silicone oil, alkyl-modified silicone, amino-modified silicone, and three-dimensional network silicone; higher fatty acids such as lauric acid, myristic acid, palmitic acid, stearic acid, isostearic acid, behenic acid, oleic acid, linoleic acid, and linolenic acid; waxes such as carnauba wax, candelilla wax, jojoba oil, beewax, and lanolin; vitamins and vitamin-like active substances; and essential oils. These may be used alone, or two or more of them may be used in combination.

Wall substance 2 has only to be any cationic polymer, which is desirably a cationic polysaccharide polymer. Examples of the material for wall substance 2 may include chitosan, gelatin, polyethylenimine, agarose, β-lactoglobulin, poly-L-lysine, and polyarginine. These may be used alone, or two or more of them may be used in combination.

In addition, the cosmetic composition according to the present exemplary embodiment includes microcapsules 10, 10a, 10b, 10c, 10d (hereinafter, referred to as microcapsules 10 in the present section) according to respective aspects as will be described later, and a water phase that disperses multiple microcapsules 10, and microcapsules 10 may have an average particle size of 3 µm ± 1 µm and a standard deviation of 0.3 ± 0.1. Thus, the core substance can be released depending on strength of external stimuli, because microcapsules 10 can range in film thickness strength of wall substance 2, that is, a wide range of microcapsules can be produced which are higher or lower in film thickness strength. For example, when microcapsules 10 are attached to hair, some microcapsules 10 have wall substances 2 broken by drying and core substance 1 released, but some other microcapsules 10 remain attached to the hair without being broken. Then, an external stimulus, that is, a stimulus such as finger combing breaks microcapsules 10 to release core substance 1.

In addition, in the cosmetic composition according to the present exemplary embodiment, a conductivity of the water phase that disperses multiple microcapsules 10 may be higher than 7.5 µS/cm and lower than 1000 µS/cm. Thus, the cosmetic composition can be electrically made into a form of a mist with hair-care device 100. In this regard, examples of hair-care device 100 may include devices that are used for drying hair or fixing hair, such as a drier and a hair iron.

In the cosmetic composition according to the present exemplary embodiment, microcapsules 10 and the water phase that disperses multiple microcapsules 10 may be equal to each other in specific gravity. Thus, microcapsules 10 are homogeneously dispersed in the water phase, and microcapsules 10 can be thus attached in a certain amount to hair on each use. In addition, even when the cosmetic composition is stored for long periods, microcapsules 10 can be kept stably dispersed without settling out.

Microcapsules 10 according to the present exemplary embodiment are classified into four aspects as will be described later, in accordance with differences among types of and between structures of wall substances 2 of microcapsules 10. In addition, cosmetic compositions containing microcapsules 10 are also classified into four aspects as will be described later, in accordance with the aspects of microcapsules 10.

The microcapsules and cosmetic compositions according to the respective aspects will be described below.

### [A-1. First aspect]

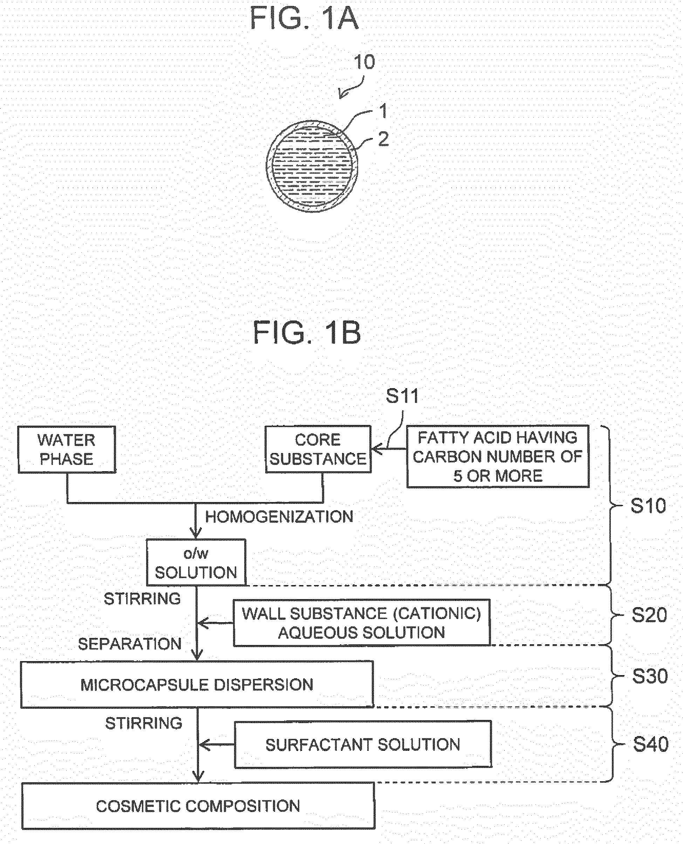

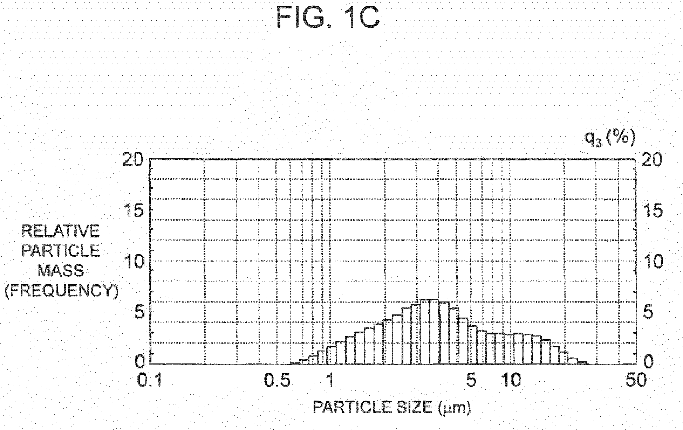

A microcapsule and a cosmetic composition according to a first aspect of the present exemplary embodiment will be described below with reference to FIGS. 1A to 1C. FIG. 1A is a schematic sectional view illustrating an example of microcapsule 10 according to the first aspect of the exemplary embodiment. FIG. 1B is a process chart showing an example of a method for producing the microcapsule and the cosmetic composition according to the first aspect of the exemplary embodiment. FIG. 1C is a graph showing results of measuring particle size distribution of the microcapsule according to the first aspect of the exemplary embodiment.

As shown in FIG. 1A, microcapsule 10 according to the first aspect is composed of core substance 1 and wall substance 2 that includes therein core substance 1. According to the present aspect, wall substance 2 includes one layer.

Microcapsule 10 and a cosmetic composition that includes microcapsule 10 according to the first aspect are produced, for example, as shown in FIG. 1B.

First, core substance 1 which is an anionic hydrophobic substance is dispersed in a water phase (for example, distilled water), thereby preparing an oil-in-water emulsion solution of core substance 1 (o/w solution) (oil-in-water emulsion solution preparation step) (S10). In this regard, the water phase of the o/w solution of core substance 1 is not limited to distilled water, and may be for example, an aqueous solution with a pH ranging from 4 to 7, inclusive, or an aqueous solution containing a lower alcohol having a carbon number of 5 or less, such as glycerol, ethanol, or isopropanol. In addition, from the viewpoint of improving solubility of respective components included in the cosmetic composition, a content of the lower alcohol having a carbon number of 5 or less in the cosmetic composition is preferably 0% by mass to 45% by mass, further preferably 0% by mass to 30% by mass, further preferably 0% by mass to 20% by mass, further preferably 0% by mass to 15% by mass, further preferably 0% by mass to 10% by mass. It is to be noted that aqueous solutions to be described later are not also limited to distilled water as is the case with the water phase.

In addition, for the dispersion, an emulsification device, a homogenizer, or the like can be used, and if necessary, a surfactant suitable for the dispersion of core substance 1 (hydrophobic substance) may be used in combination.

Further, in step S10, in order to adjust the anionic strength of core substance 1, a fatty acid having a carbon number of 5 or more may be added (fatty acid addition step) (S11). In step S11, when the fatty acid having a carbon number of 5 or more, contained in core substance 1, is less than 80% in percent by weight, the fatty acid may be added such that the fatty acid contained in core substance 1 is 80% or more. As long as the fatty acid having a carbon number of 5 or more, contained in core substance 1, is 80% or more, microcapsule 10 can be obtained which has a desired film thickness. In this regard, examples of the fatty acid having a carbon number of 5 or more may include a hexanoic acid, a heptanoic acid, an octanoic acid, a lauric acid, a stearic acid, a linoleic acid, and an oleic acid. These may be used alone, or two or more of them may be used in combination.

In addition, in step S10, it is possible to adjust the particle size of a finally obtained microcapsule by adjusting the particle size of core substance 1 in the oil-in-water emulsion solution (o/w solution) of core substance 1.

Next, while stirring the oil-in-water emulsion solution (o/w solution) obtained in step S10, an aqueous solution of wall substance 2 which is a cationic polymer (the material herein is a dilute acid solution of chitosan) is added to and mixed with the oil-in-water emulsion solution (inclusion step) (S20), and a microcapsule dispersion is then separated through use of a separating funnel (S30). Thus, the microcapsule dispersion can be obtained in which microcapsule 10 according to the first aspect is dispersed.

Subsequently, a surfactant solution is added to the microcapsule dispersion obtained in step S30, thereby adjusting a conductivity (electrical conductivity) of the water phase of the microcapsule dispersion (S40). Thus, the cosmetic composition according to the first aspect can be obtained.

In this regard, the particle size of the finally obtained microcapsule may be smaller than 200 µm, and from the viewpoint of storage stability and usability, ranges from preferably 0.5 µm to 50 µm, inclusive, more preferably 0.5 µm to 30 µm, inclusive. Since a mist discharge outlet port of a hair-care device to be described later is 200 µm in hole diameter, it is possible to make a cosmetic composition containing the microcapsule into a form of a mist (electrostatic atomization) in an appropriate manner with use of the hair-care device, as long as the microcapsule is smaller than 200 µm in particle size. In addition, even when hair is touched with a hand after the microcapsule is attached to the hair, there is no feeling of strangeness such as stickiness, but good usability such as affinity to hair is obtained. It is to be noted that the foregoing particle size ranges are derived from FIG. 1C and a sensory evaluation to be described later. As shown in FIG. 1C, the microcapsule has a particle size distribution in the range from 0.5 µm to 30 µm. It is to be noted that the particle size and particle size distribution of the microcapsule were measured with use of a laser diffraction-type particle size distribution measurement system.

In the case of electrically making the cosmetic composition mentioned above into the form of a mist with use of the hair-care device to be described later, a conductivity of the water phase of the cosmetic composition may be higher than 7.5 µS/cm and lower than 1000 µS/cm. Therefore, in the method for producing a cosmetic composition according to the present disclosure, a known surfactant may be added appropriately to adjust the conductivity (electrical conductivity), as in step S40. It is to be noted that description of the hair-care device here is omitted, because the hair-care device will be described later.

In addition, microcapsule 10 contained in the cosmetic composition mentioned above is formed by a heteroaggregation method for electrostatically aggregating anionic core substance 1 and cationic wall substance 2. In order for microcapsule 10 according to the first aspect to be broken by a desired stimulus at desired timing to release core substance 1, wall substance 2 may be adjusted to desired hardness.

In this regard, the desired timing refers to, for example, after the microcapsule is attached to hair, and the desired stimulus refers to, for example, a stimulus such as a pressure due to finger combing, a friction, a wind pressure, or a temperature, in the case of drying hair.

Examples of a method for adjusting wall substance 2 of microcapsule 10 to the desired hardness may include a method of adjusting a film thickness of wall substance 2 by adjusting the anionic strength of core substance 1, a method of making wall substance 2 cross-linked with a cross-linking agent, and a method of dehydrating wall substance 2 with use of a poor solvent.

The cross-linking agent is not particularly limited as long as wall substance 2 can be made cross-linked by the agent, and examples of the agent include genipin and tripolyphosphoric acid. In addition, the poor solvent is not particularly limited as long as wall substance 2 can be dehydrated, and examples of the solvent include sodium carbonate and dehydrated alcohol.

### [A-2. Second aspect]

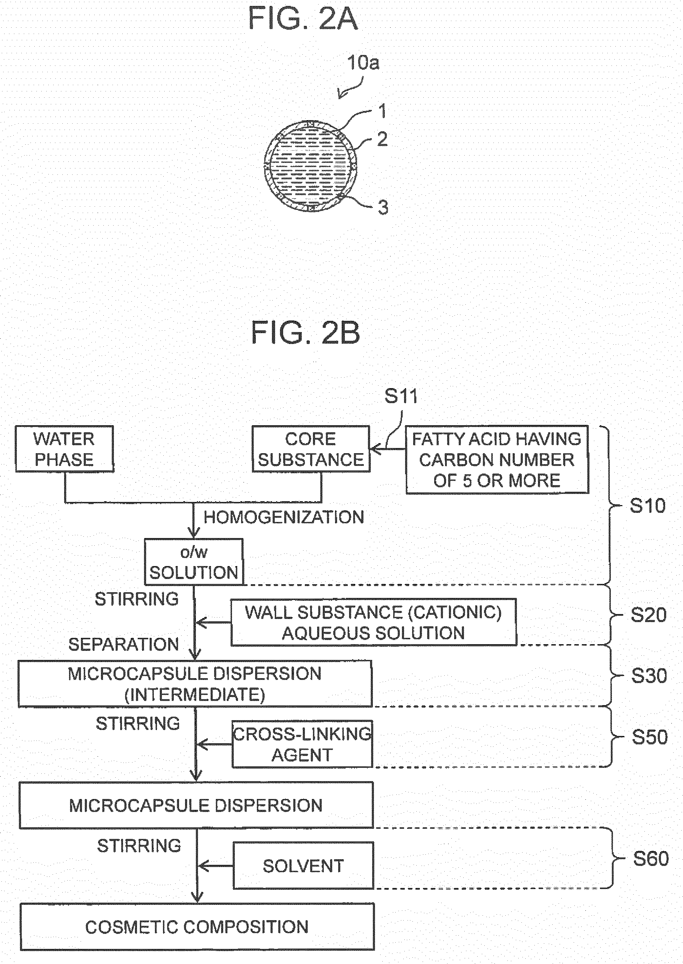

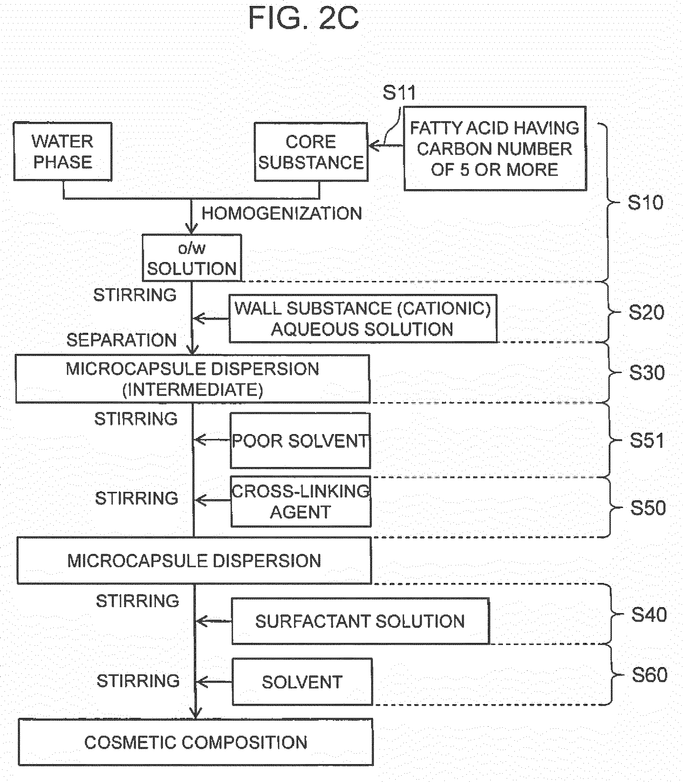

A microcapsule and a cosmetic composition according to a second aspect of the present exemplary embodiment will be described below with reference to FIGS. 2A to 2C. FIG. 2A is a schematic sectional view illustrating an example of the microcapsule according to the second aspect of the exemplary embodiment. FIG. 2B is a process chart showing an example of a method for producing the microcapsule and the cosmetic composition according to the second aspect of the exemplary embodiment. FIG. 2C is a process chart showing another example of a method for producing the microcapsule and the cosmetic composition according to the second aspect of the exemplary embodiment.

As shown in FIG. 2A, microcapsule 10a according to the second aspect is composed of core substance 1 and wall substance 2 that includes therein core substance 1. According to the second aspect, wall substance 2 has cross-links 3 formed with cross-linking agent. A degree of cross-linkage for wall substance 2 is adjusted, thereby making it possible to improve the film thickness strength, and thus making it possible to obtain microcapsule 10a which has desired film thickness strength. Specific examples of the cross-linking agent are provided as described above, and description of the agent here is thus omitted.

Microcapsule 10a and a cosmetic composition containing microcapsule 10a according to the second aspect are produced, for example, as shown in FIG. 2B. It is to be noted that repeated description of the first aspect will be hereinafter omitted.

First, core substance 1 which is an anionic hydrophobic substance is dispersed in a water phase (for example, distilled water), thereby preparing an oil-in-water emulsion solution of core substance 1 (o/w solution) (oil-in-water emulsion solution preparation step) (S10). Further, in step S10, in order to adjust the anionic strength of core substance 1, a fatty acid having a carbon number of 5 or more may be added (fatty acid addition step) (S11).

Next, while stirring the oil-in-water emulsion solution (o/w solution) obtained in step S10, an aqueous solution of wall substance 2 which is a cationic polymer (the material herein is a dilute acid solution of chitosan) is added to and mixed with the oil-in-water emulsion solution (inclusion step) (S20), and a microcapsule dispersion (intermediate) is then separated through use of a separating funnel (S30).

Subsequently, an aqueous solution of a cross-linking agent is added to the microcapsule dispersion (intermediate) obtained in step S30, thereby adjusting wall substance 2 of microcapsule 10 to desired hardness (cross-linking step) (S50). Thus, the microcapsule dispersion can be obtained in which microcapsule 10a according to the second aspect is dispersed.

Subsequently, the microcapsule dispersion obtained in step S50 is stirred with addition of solvent thereto (S60). It is to be noted that the solvent may be the same as the water phase described above, or a lower alcohol having a carbon number of 5 or less. Thus, the cosmetic composition according to the second aspect can be obtained.

Alternatively, microcapsule 10a and the cosmetic composition containing microcapsule 10a according to the second aspect may be produced, for example, as shown in FIG. 2C. Steps that differ from the steps in FIG. 2B will be described here.

In step S30, the microcapsule dispersion (intermediate) is separated with use of a separating funnel. While stirring the microcapsule dispersion (intermediate) obtained, a poor solvent is added to the microcapsule dispersion (intermediate), thereby dehydrating wall substance 2 of microcapsule 10a (dehydration step) (S51). In step S51, of wall substance 2 constituting a coating part of microcapsule 10a, a surface in contact with the poor solvent is dehydrated. Thus, the hardness of wall substance 2 of microcapsule 10a can be adjusted. It is to be noted that specific examples of the poor solvent have been described above, and description of the solvent here is thus omitted.

Subsequently, an aqueous solution of a cross-linking agent is added to the microcapsule dispersion (intermediate) obtained in step 51, thereby making wall substance 2 of microcapsule 10a cross-linked (cross-linking step) (S50). As just described, wall substance 2 of microcapsule 10a can be adjusted to desired hardness by adjusting the degree of cross-linkage for wall substance 2. In accordance with the cross-linking step (S50), a microcapsule dispersion can be obtained in which microcapsule 10a according to another example of the second aspect is dispersed.

Subsequently, a surfactant solution is added to the microcapsule dispersion obtained in step S50, thereby adjusting a conductivity (electrical conductivity) of the water phase of the microcapsule dispersion (S40). Finally, the microcapsule dispersion obtained in step S40 is stirred with addition of a solvent (for example, distilled water) thereto (S60). Thus, a cosmetic composition according to another example of the second aspect can be obtained.

### [A-3. Third aspect]

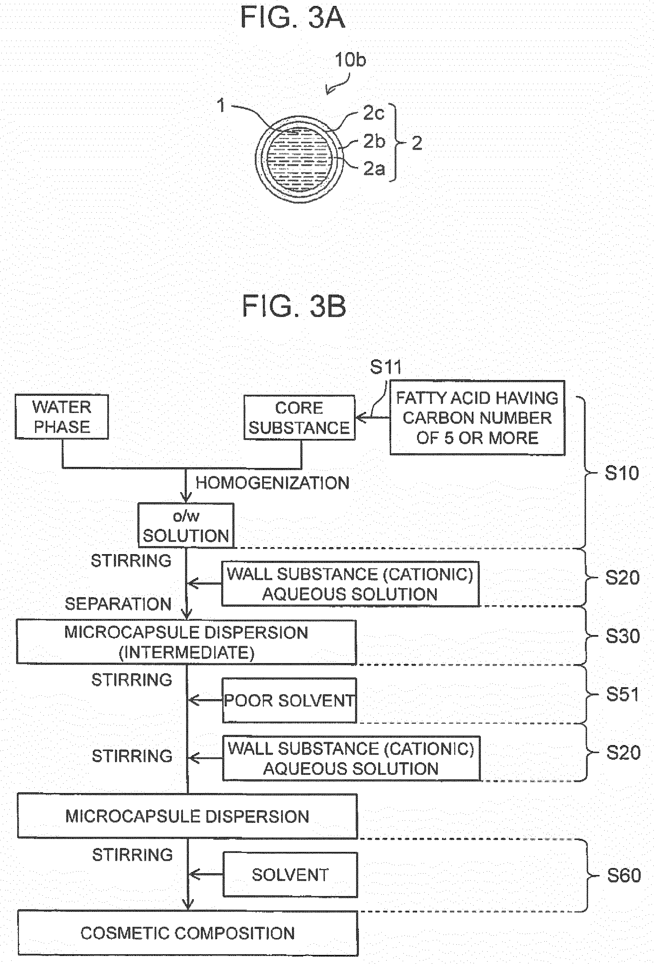

A microcapsule and a cosmetic composition according to a third aspect of the present exemplary embodiment will be described with reference to FIGS. 3A and 3B. FIG. 3A is a schematic sectional view illustrating an example of the microcapsule according to the third aspect of the exemplary embodiment. FIG. 3B is a process chart showing an example of a method for producing the microcapsule and the cosmetic composition according to the third aspect of the exemplary embodiment.

As shown in FIG. 3A, microcapsule 10b according to the third aspect is composed of core substance 1 and wall substance 2 that includes therein core substance 1. According to the present aspect, wall substance 2 includes, for example, a plurality of layers (a layer of wall substance 2a and a layer of wall substance 2b). The plurality of layers (the layer of wall substance 2a and the layer of wall substance 2b) may be composed of wall substances 2 which are identical cationic polymers, or composed of wall substances 2a and 2b which are different cationic polymers. According to the present aspect, dehydrated layer 2c (a layer of a dehydrated surface of wall substance 2a) is formed between the plurality of layers (the layer of wall substance 2a and the layer of wall substance 2b). As just described, wall substance 2 includes the plurality of layers (the layer of wall substance 2a, the layer of wall substance 2b, and dehydrated layer 2c), thereby making it possible to improve the film thickness strength of wall substance 2, and thus making it possible to obtain microcapsule 10b which has desired film thickness strength.

Microcapsule 10b and a cosmetic composition containing microcapsule 10b according to the third aspect are produced, for example, as shown in FIG. 3B. It is to be noted that repeated description of the first and second aspects will be hereinafter omitted.

While stirring a microcapsule dispersion (intermediate) separated with use of a separating funnel in step S30, a poor solvent is added to the dispersion, thereby dehydrating a surface of wall substance 2a of microcapsule 10b (dehydration step) (S51). In step S51, of wall substance 2a constituting wall substance 2 of microcapsule 10b, a surface in contact with the poor solvent is dehydrated. Thus, a degree of cure for the surface of the layer of wall substance 2a can be adjusted, and the film thickness strength of wall substance 2 can be thus improved. It is to be noted that specific examples of the poor solvent have been described above, and description of the solvent here is thus omitted.

Next, while stirring the microcapsule dispersion obtained in step S51, wall substance 2 (the material herein is a dilute acid solution of chitosan) which is a cationic polysaccharide polymer is added to and mixed with the dispersion, thereby further forming the layer of wall substance 2b on the surface of wall substance 2a, which is dehydrated in step S51 (inclusion step) (S20). Thus, the microcapsule dispersion can be obtained in which microcapsule 10b according to the third aspect is dispersed.

Subsequently, the obtained microcapsule dispersion is stirred with addition of a solvent (for example, distilled water) thereto (S60). Thus, the cosmetic composition according to the third aspect can be obtained.

### [A-4. Fourth aspect]

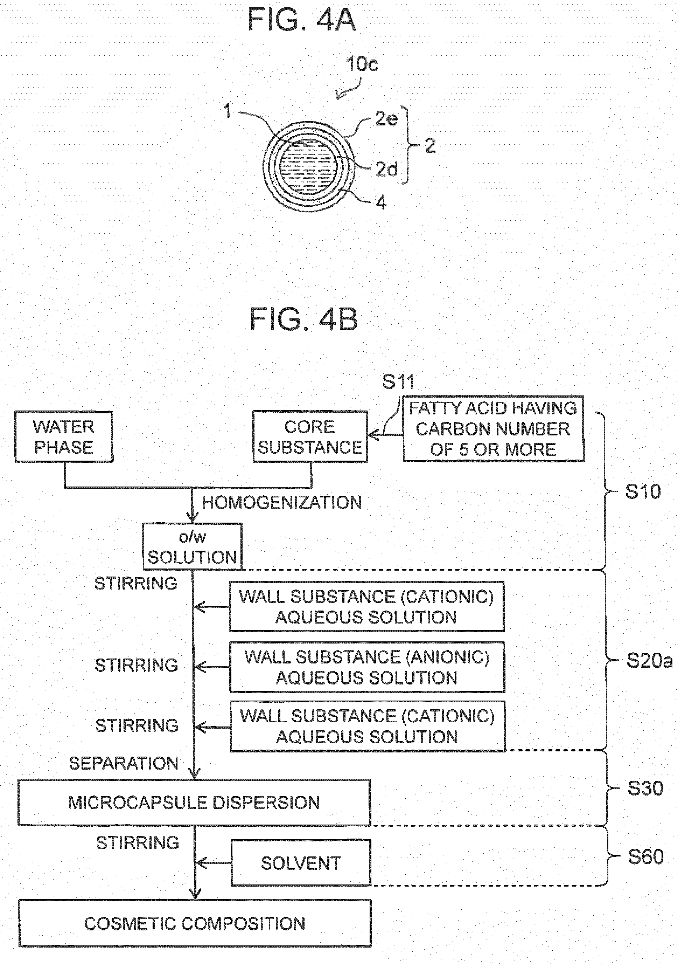

A microcapsule and a cosmetic composition according to the fourth aspect of the present exemplary embodiment will be described below with reference to FIGS. 4A and 4B. FIG. 4A is a schematic sectional view illustrating an example of the microcapsule according to the fourth aspect of the exemplary embodiment. FIG. 4B is a process chart showing an example of a method for producing the microcapsule and the cosmetic composition according to the fourth aspect of the exemplary embodiment.

As shown in FIG. 4A, microcapsule 10c according to the fourth aspect is composed of core substance 1 and wall substance 2 that includes therein core substance 1. According to the present aspect, wall substance 2 includes, for example, a plurality of layers. When wall substance 2 includes a plurality of layers, the outermost layer has only to be cationic, and for example, of the plurality of layers constituting wall substance 2, a layer of cationic wall substance 2d and a layer of cationic wall substance 2e may be disposed in order of distance from core substance 1. Furthermore, a layer of anionic wall substance 4 may be disposed between cationic wall substances 2d and 2e. According to the present aspect, for example, wall substances 2d, 2e, and 4 are all polysaccharide polymers.

Anionic wall substance 4 has only to be an anionic polymer, which is desirably an anionic polysaccharide polymer. Examples of the material for wall substance 4 may include pectic acid, alginic acid, agarose, agar, carrageenan, fucoidan, hyaluronic acid, chondroitin sulfate, heparin, gum arabic, casein sodium, gellan gum, native gellan gum, xanthan gum, carboxymethyl cellulose, carboxymethyl starch, and carboxymethyl dextran. These may be used alone, or two or more of them may be used in combination.

Microcapsule 10c and a cosmetic composition containing microcapsule 10c according to the fourth aspect are produced, for example, as shown in FIG. 4B. It is to be noted that repeated description of the first to third aspects will be hereinafter omitted.

While stirring the oil-in-water emulsion solution (o/w solution) obtained in step S10, an aqueous solution of wall substance 2d which is a cationic polymer (the material herein is a dilute acid solution of chitosan) is added to and mixed with the oil-in-water emulsion solution, thereby forming the layer of cationic wall substance 2d, which includes therein core substance 1. Subsequently, an aqueous solution of wall substance 4 which is an anionic polymer (the material herein is an aqueous solution of gum arabic) is added and mixed, thereby forming the layer of anionic wall substance 4, which coats the layer of cationic wall substance 2d. Furthermore, an aqueous solution of wall substance 2e which is a cationic polymer (the material herein is a dilute acid solution of chitosan) is added and mixed, thereby forming the layer of cationic wall substance 2e, which coats the layer of anionic wall substance 4 (inclusion step) (S20a). In this regard, as described above in the third aspect, the materials for cationic wall substances 2d and 2e may be identical cationic polymers, or may be different cationic polymers.

Subsequently, after the addition of a water phase (for example, distilled water), the microcapsule dispersion is separated with use of a separating funnel (S30). Thus, the microcapsule dispersion can be obtained in which microcapsule 10c according to the fourth aspect is dispersed.

Then, the cosmetic composition according to the fourth aspect can be obtained through addition of a solvent (distilled water herein) to the obtained microcapsule dispersion.

### [B. Hair-care device]

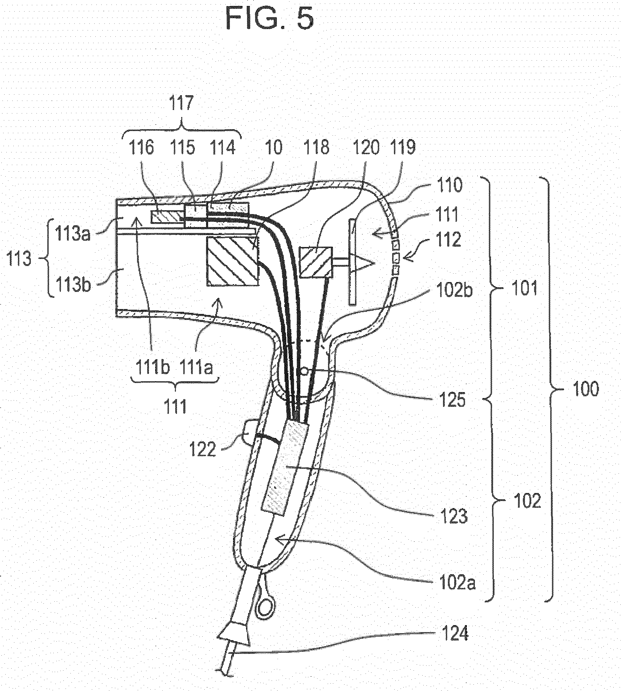

The cosmetic composition described above is used through introduction of the cosmetic composition into a hair-care device FIG. 5 is a schematic configuration diagram illustrating an example of hair-care device 100 that includes a cosmetic composition according to the present disclosure.

A cosmetic composition including microcapsule 10 described above is inserted into tank part 114 of hair-care device 100 shown in FIG. 5.

In FIG. 5, main body section 101 of hair care device 100 includes housing 110 constituting a framework, and housing 110 is composed of multiple divided parts joined together.

Housing 110 has therein hollow 111 formed, and in hollow 111, blowing flow channel 111a is formed from inlet port 112 on one side (right side of FIG. 5) in a longitudinal direction (horizontal direction of FIG. 5) to outlet port 113 on another side (left side of FIG. 5), and various types of electrical parts are housed.

More specifically, the blowing flow channel provided with inlet port 112 and outlet port 113 at both ends in the longitudinal direction is provided inside housing 110 of main body section 101.

A lower part of housing 110 has grasping section 102 as a part held with a user's hand, which is bonded in a direction that intersects with the longitudinal direction of main body section 101, and configured such that main body section 101 and grasping section 102 have a substantially T-shaped or substantially L-shaped (substantially T-shaped according to the present exemplary embodiment) appearance in use of hair-care device 100.

A side surface of grasping section 102 is provided with sliding power switch 122, and power cord 124 is extended from a protruding end (distal end 102a) of grasping section 102. In addition, grasping section 102 with base 102b closer to main body section 101, and distal end 102a, is rotatably connected to main body section 101 via connection 125 at base 102b. Thus, hair-care device 100 can be folded to bring distal end 102a into a position along main body section 101.

On the other hand, fan 119 and motor 120 as blowing part 121 are disposed upstream (closer to inlet port 112) in blowing flow channel 111a mentioned above.

Heater 118 as a heating part for heating blowing from fan 119 is disposed downstream of fan 119 (on the side closer to outlet port 113 than fan 119). Heater 118 is formed, for example, by winding and disposing a band-shaped and corrugated electrical resistor around an inner circumference of blowing flow channel 111a.

In addition, discharger 116 provided with a discharge electrode for electrostatically atomizing a liquid supplied to produce a liquid of charged microparticles, pump 115 as a liquid supply for supplying a liquid to discharger 116, and tank 114 as a liquid reservoir are housed in branched flow channel 111b provided separately from blowing flow channel 111a above blowing flow channel 111a.

In this regard, a main constituent of the discharge electrode is composed of brass, and a hole through which the microcapsule dispersion passes is appropriately made in a center of the brass. The cosmetic composition inserted into tank 114 is supplied by increments through pump 115, and supplied to the discharge electrode through the hole of the brass.

Furthermore, controller 123 for controlling each of electric power supplied to motor 120 and electric power supplied to heater 118 is provided on a side of main body section 101 closer to inlet port 112. When a voltage is applied to the discharge electrode by supplying electric power to discharger 116 (applying electric current to the discharger according to the present exemplary embodiment), the cosmetic composition supplied to the discharge electrode is made into a form of a mist (atomized) by energy of discharge generated between the discharge electrode and a ground electrode disposed downstream of the discharge electrode in branched flow channel 111b.

The produced mist of the cosmetic composition is discharged to outside from mist discharge outlet port 113a provided at a head of branched flow channel 111b, and then supplied to user's hair along with blowing discharged from hot air discharge outlet port 113b.

Operation and behavior of hair-care device 100 (for example, drier) configured as described above will be described.

When a user turns on sliding power switch 122, electric power is supplied through power cord 124 to controller 123, thereby driving controller 123.

Controller 123 driven applies electric current to pump part 115 and discharger 116. The cosmetic composition inserted into tank 114 is supplied by increments to discharger 116 through supply of the electric current to pump part 115. On the other hand, controller 123 applies a voltage to the discharge electrode to cause discharge between the discharge electrode and the ground electrode, thereby making the cosmetic composition finer into the form of a mist.

On the other hand, when power switch 122 is turned on, electric power is supplied to motor 120 and heater 118 to rotary-drive motor 120, thereby rotating fan 119, and thus forming an airflow. The airflow is formed in a way that air flowing from outside through inlet port 112 into blowing flow channel 111a is discharged through blowing flow channel 111a from outlet port 113 again to the outside. Also, heater 118 is driven to heat the air blown by fan 119, and the heated air is blown as hot air from hot air discharge outlet port 113b to the outside, and supplied to the user's hair. In this regard, the mist of the cosmetic composition, discharged from mist discharge outlet port 113a to the outside, is supplied the user's hair by currents of the airflow discharged from outlet port 113 to the outside as described above.

Microcapsule 10 contained in the cosmetic composition attached to the hair has wall substance 2 broken by various stimuli such as drying, a pressure due to finger combing, a temperature, or a wind pressure. When wall substance 2 is broken, core substance 1 acts on the hair to produce a hair-care effect. Thereafter, when the user turns off power switch 122, power supply to controller 123 is stopped, thereby also stopping electric current flowing through pump part 115 and discharger 116, and as a result, stopping the generation of the mist. Furthermore, power supply to fan 119 and motor 120 is also stopped, thereby causing hair-care device 100 to stop operation.

### EXAMPLES

Hereinafter, the present disclosure will be more specifically described with reference to examples, but the present disclosure is not to be considered limited to the following examples.

### [Production of microcapsule]

In the present example, a camellia oil was used as a core substance of a microcapsule, and chitosan was used as a wall substance including therein the core substance. The method for producing the microcapsule was provided in accordance with the flow shown in FIG. 1B.

First, 20 g of distilled water, 2 g of the camellia oil, and furthermore, 0.2 g of an oleic acid were added into a beaker (S11), and stirred at 15000 rpm in a homogenizer, thereby providing an oil-in-water emulsion solution (o/w solution) of the camellia oil (S10). While stirring the obtained o/w solution, a citric acid aqueous solution of the chitosan (chitosan content: 2% by mass) was mixed with the o/w solution (S20). Thereafter, the oils which had failed to be capsulated (the camellia oil and the oleic acid here) was separated and removed through a separating funnel, thereby providing a microcapsule dispersion (S30).

### [Preparation of cosmetic composition]

To the microcapsule dispersion obtained, 0.1% by mass of Tween (registered trademark) 80 and 0.1% by mass of QUARTAMIN (registered trademark) 24P were added, thereby adjusting the conductivity of the water phase of the cosmetic composition (S40). In this way, five types of cosmetic compositions were obtained where the conductivities (electrical conductivities) were respectively adjusted to 7.5 µS/cm, 33.8 µS/cm, 29.3 µS/cm, 777 µS/cm, and 1991 µS/cm.

### [Particle Size Measurement of Microcapsule]

The average particle size was measured for the microcapsules included in the cosmetic compositions obtained by the method mentioned above. The result is shown in FIG. 1C.

As shown in FIG. 1C, the average particle size for the microcapsules was 3.69 µm in median diameter and 3.60 µm in mode diameter, the standard deviation was 0.33, and the CV value was 2.07. Thus, it has been successfully confirmed that the cosmetic compositions obtained by the method mentioned above fall within the range of 3 µm ± 1 µm in average particle size, and within the range of 0.3 ± 0.1 in standard deviation.

### [Confirmation test for atomization with difference in electrical conductivity]

The method described above was adopted to prepare five types of cosmetic compositions where the conductivities (electrical conductivities) of the water phases of the cosmetic compositions were respectively adjusted to 7.5 µS/cm, 33.8 µS/cm, 293 µS/cm, 777 µS/cm, and 1991 µS/cm. Then, these cosmetic compositions were each discharged at a pumping rate of 0.2 ml/h, a voltage of 3 kV to 5 kV was applied to a discharge electrode, and the atomization was confirmed. As for flying atomized particles, a glass plate was disposed to be opposed to a head of the discharge electrode (on the side with atomized particles discharged), and whether the atomized particles were attached to the surface of the glass plate or not was visually confirmed.

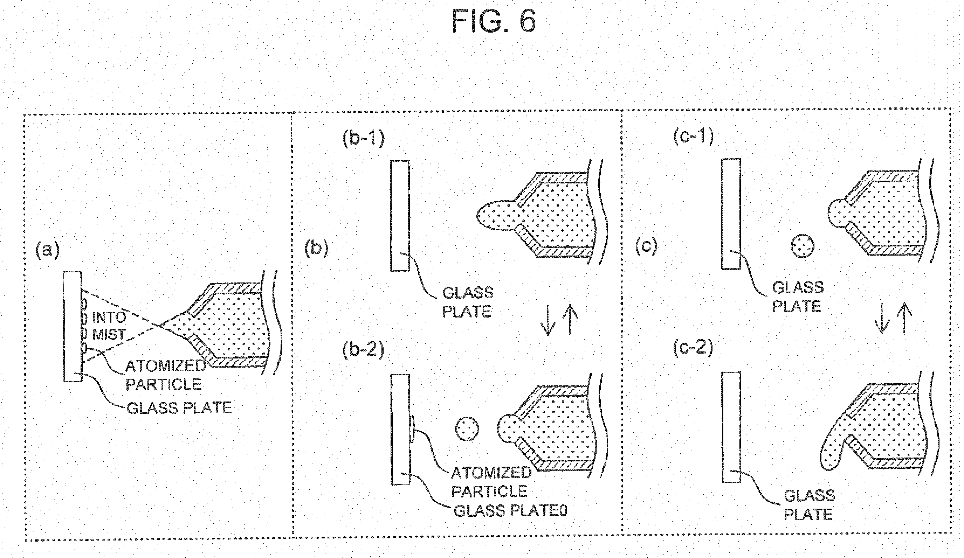

The atomization of the cosmetic composition was confirmed by determining whether any Taylor corn is formed or not. Specifically, as shown in FIGS. 6A through 6C-2, a predetermined voltage was applied to the discharge electrode, and whether the cosmetic composition was electrostatically atomized successfully was confirmed, and whether the cosmetic composition was stably atomized successfully was confirmed.

As shown in part (a) of FIG. 6, when a conical Taylor corn was confirmed successfully at the head of the discharge electrode, and when the attachment of the atomized cosmetic composition to the surface of the glass plate was continuously confirmed successfully, it was determined that atomized particles (mist) were stably sprayed (○). In addition, as shown part (b) in FIG. 6, when the state of (b-1) and the state of (b-2) were alternately repeated without stably forming a conical Taylor corn at the head of the discharge electrode, and when the attachment of atomized particles to the surface of the glass plate was confirmed irregularly, it was determined that the atomized particles were unstably sprayed (Δ). In addition, as shown in part (c) of FIGS. 6, when the state of (c-1) and the state of (c-2) were alternately repeated without forming a Taylor corn at the head of the discharge electrode, and when the attachment of atomized particles to the surface of the glass plate was not confirmed successfully, it was determined that atomized particles were not formed (×). The results are shown in Table 1.

**[Table 1]**

| Electrical conductivity (µS/cm) | Applied voltage (kv) | | | | |
|---|---|---|---|---|---|
| | 3 | 3.5 | 4 | 4.5 | 5 |
| 7.5 | × | Δ | ○ | ○ | ○ |
| 33.8 | Δ | ○ | ○ | ○ | ○ |
| 293 | Δ | ○ | ○ | ○ | ○ |
| 777 | Δ | Δ | ○ | ○ | ○ |
| 1991 | × | × | Δ | Δ | Δ |

As shown in Table 1, at the applied voltage of 3.5 kV, when the electrical conductivity (conductivity) of the water phase of the cosmetic composition was 7.5 µS/cm and 777 µS/cm, atomized particles were unstably sprayed, and when the conductivity was 1991 µS/cm, the cosmetic composition was not electrostatically atomized successfully.

At the applied voltage of 4 kV, when the electrical conductivity (conductivity) of the water phase of the cosmetic composition was 7.5 µS/cm and 777 µS/cm, atomized particles were stably sprayed, but when the conductivity was 1991 µS/cm, atomized particles were unstably sprayed.

Accordingly, it was determined that for the cosmetic composition, when the applied voltage applied to the discharge electrode of the hair-care device is adjusted to 4 kV ± 0.5 kV, the electrical conductivity of the water phase has only to be higher than 7.5 µS/cm and lower than 1000 µS/cm.

### [Sensory evaluation]

For six panels, Example 1, Comparative Example 1, and Comparative Example 2 were evaluated for the following seven items (shininess, cohesiveness, volume control, moisture, smoothness, finger combing, and softness of hair).

In Example 1, the cosmetic composition according to the present disclosure was attached to hair with a hair drier (atomization drier) as in FIG. 5. In Comparative Example 1, a conventional drier was used without applying anything to hair. In Comparative Example 2, a conventional drier was used after applying the core substance (camellia oil here) to hair.

Evaluations were conducted on the following criteria.
1: bad, 2: somewhat bad, 3: normal, 4: somewhat good, 5: good

The results are shown in Table 2. It is to be noted that the score for each item refers to the average of points for the six panels.

**[Table 2]**

| | | Evaluation item | | | | | | | Comprehensive |
|---|---|---|---|---|---|---|---|---|---|
| | | Shininess | Cohesiveness | Volume control | Moisture | Smoothness | Finger combing | Softness | |
| Example 1 | Cosmetic composition according to present disclosure + Atomization drier (FIG. 5) | 3.54 | 4.13 | 3.74 | 4.05 | 4.00 | 4.46 | 3.88 | 4.34 |
| Comparative Example 1 | Conventional drier | 3.00 | 3.50 | 3.50 | 3.00 | 3.08 | 3.42 | 3.42 | 3.17 |
| Comparative Example 2 | Core substance application + Conventional drier | 3.33 | 3.67 | 3.50 | 3.67 | 3.00 | 3.50 | 3.25 | 3.67 |

As shown in Table 2, in Example 1, the greatest hair-care effects have been achieved for all of the items. Next, Comparative Example 2 has achieved a great hair-care effect in the comprehensive evaluation. According to Comparative Example 2, the application of the core substance to the hair has achieved relatively great effects with respect to the four items of shininess, cohesiveness, moisture, and finger combing of hair, but the worst results among the evaluation targets with respect to the two items of smoothness and softness of hair. In Comparative Example 2, it was difficult to apply the core substance uniformly to the hair, thereby producing unevenness of coating. Therefore, the smoothness and softness of the hair are considered to have been decreased.

From the foregoing results, it was determined that the shininess, cohesiveness, volume control, moisture, and finger combing of hair are improved in each case of Example 1 and Comparative Example 2, as compared with Comparative Example 1. More specifically, it was determined that the attachment of the core substance to the hair surface achieves the effects mentioned above.

On the other hand, with respect to the smoothness and softness of the hair, Example 1 has achieved favorable results, but Comparative Example 2 has the worst results, among the three evaluation targets mentioned above. As described above, in Comparative Example 2, it was difficult to apply the core substance uniformly to the hair, thereby producing unevenness of coating. Therefore, the smoothness and softness of the hair were decreased. In Example 1, the cosmetic composition including the microcapsulated core substance is atomized and attached to hair, and it is thus believed that the core substance can be uniformly attached to hair in a smaller amount than in Comparative Example 2. In addition, in Example 1, the core substance included in the cosmetic composition is microcapsulated, and it is thus believed that the core substance is made more likely to penetrate into hair, as compared with a case of applying the core substance to hair as in Comparative Example 2. As just described, it was determined that in Example 1, the hair-care characteristics of the core substance can be maximized by not only uniformly attaching the core substance to hair surfaces, but also making the core substance into a formulation which makes the core substance likely to penetrate into the hair.

Accordingly, it was successfully confirmed that when the cosmetic composition containing the microcapsulated core substance is atomized (made into form of a mist), and attached to hair, a greater hair-care effect is achieved than in the case of applying the core substance to hair.

### (Summary)

As described above, microcapsules 10 and 10a to 10d (hereinafter, referred to as 10 and the like) according to the exemplary embodiment mentioned above refer to microcapsules 10 and the like each composed of core substance 1 and wall substance 2 including core substance 1 in wall substance 2, where core substance 1 is an anionic hydrophobic substance, wall substance 2 is a cationic polymer, and microcapsules 10 and the like are smaller than 200 µm in particle size. Thus, microcapsules 10 and the like can be electrostatically formed by a difference in surface charge between anionic core substance 1 and cationic wall substance 2. In addition, thicknesses of wall substances 2 of microcapsules 10 and the like can be adjusted depending on anionic strength of core substances 1. Thus, microcapsules 10 and the like can be adjusted to desired strength. In addition, since mist discharge outlet port 113a of hair-care device 100 described above is 200 µm in hole diameter, it is possible to make a cosmetic compositions containing microcapsules 10 and the like into the form of a mist (electrostatic atomization) in an appropriate manner with use of hair-care device 100, as long as microcapsules 10 and the like are smaller than 200 µm in particle size. Furthermore, even when hair is touched with a hand after microcapsules 10 and the like are attached to the hair, there is no feeling of strangeness such as stickiness, but good usability such as affinity to hair is obtained.

In microcapsules 10 and the like according to the exemplary embodiment mentioned above, core substance 1 has a form of a liquid. Thus, core substance 1 is likely to be released from inside of microcapsules 10 and the like when microcapsules 10 and the like are broken. In addition, core substance 1 in the form of a liquid is thus easily applied to hair.

In microcapsules 10 and the like according to the exemplary embodiment mentioned above, core substance 1 contains a fatty acid having a carbon number of 5 or more at 80% or more in percent by weight. Thus, microcapsules 10 and the like can be obtained which have a desired film thickness.

In microcapsule 10a according to the first aspect of the exemplary embodiment, wall substance 2 is cross-linked. As just described, the film thickness strength of wall substance 2 of microcapsule 10a can be improved by adjusting the degree of cross-linkage for wall substance 2. Thus, microcapsule 10a can be obtained which have desired film thickness strength.

In microcapsules 10b to 10d according to the second to fourth aspects of the exemplary embodiment, wall substance 2 is composed of a plurality of layers. As just described, wall substances 2 form coating films composed of a plurality of layers, thereby making it possible to improve the film thickness strength of wall substances 2 of microcapsules 10b to 10d. Thus, microcapsules 10b to 10d can be obtained which have desired film thickness strength.

The cosmetic composition according to the exemplary embodiment mentioned above includes a plurality of microcapsules 10 and the like as mentioned above, and a water phase that disperses the plurality of microcapsules 10 and the like, and the plurality of microcapsules 10 and the like is 3 µm ± 1 µm in average particle size, and 0.3 ± 0.1 in standard deviation. Thus, core substance 1 can be released depending on the strength of external stimuli, because microcapsules 10 and the like can range in film thickness strength of wall substance 2.

In the cosmetic composition according to the exemplary embodiment mentioned above, the water phase has a conductivity of higher than 7.5 µS/cm and lower than 1000 µS/cm. Thus, the cosmetic composition can be electrically made into a form of a mist with hair-care device 100.

In the cosmetic composition according to the exemplary embodiment mentioned above, microcapsules 10 and the like are equal to the water phase in specific gravity. Thus, microcapsules 10 and the like are homogeneously dispersed in the water phase, and microcapsules 10 and the like can be thus attached in certain amounts to hair on each use. In addition, even when the cosmetic composition is stored for long periods, microcapsule 10 and the like can be kept stably dispersed without settling out.

The method for producing microcapsules 10 and the like according to the exemplary embodiment mentioned above refers to a method for producing a microcapsule composed of core substance 1 and wall substance 2 including core substance 1 in wall substance 2, the method includes: an oil-in-water emulsion solution preparation step of preparing an oil-in-water emulsion solution of core substance 1 which is an anionic hydrophobic substance by dispersing, in a water phase, core substance 1; and an inclusion step of including, in wall substance 2 which is a cationic polymer, microparticles of core substance 1 in the oil-in-water emulsion solution by mixing the oil-in-water emulsion solution with wall substance 2, and microcapsules 10 and the like are smaller than 200 µm in particle size. Thus, microcapsules 10 and the like can be electrostatically formed by a difference in surface charge between anionic core substance 1 and cationic wall substance 2. In addition, thicknesses of wall substances 2 of microcapsules 10 and the like can be adjusted depending on anionic strength of core substances 1. Thus, microcapsules 10 and the like can be adjusted to desired strength. In addition, since mist discharge outlet 113a of hair-care device 100 described above is 200 µm in hole diameter, it is possible to make a cosmetic compositions containing microcapsules 10 and the like into a form of a mist (electrostatic atomization) in an appropriate manner with use of hair-care device 100, as long as microcapsules 10 and the like are smaller than 200 µm in particle size. Furthermore, even when hair is touched with a hand after microcapsules 10 and the like are attached to the hair, there is no feeling of strangeness such as stickiness, but good usability such as affinity to hair is obtained.

In the method for producing microcapsules 10 and the like according to the exemplary embodiment mentioned above, the oil-in-water emulsion solution preparation step further includes a fatty acid addition step of adding, to core substance 1, a fatty acid having a carbon number of 5 or more. Thus, microcapsules 10 and the like can be obtained which have a desired film thickness.

The method for producing microcapsules 10 and the like according to the exemplary embodiment mentioned above further has a cross-linking step of making wall substance 2 cross-linked. As just described, a degree of cross-linkage for wall substance 2 of microcapsules 10 and the like is adjusted, thereby making it possible to improve the film thickness strength, and thus making it possible to obtain microcapsule 10 and the like which have desired film thickness strength.

The method for producing microcapsules 10 and the like according to the exemplary embodiment mentioned above further has, after the inclusion step, a dehydration step of dehydrating wall substance 2 of microcapsules 10 and the like with a poor solvent. Thus, a degree of cure for the surfaces of wall substances 2 of microcapsules 10 and the like can be adjusted, and the film thickness strength can be thus improved.

The method for producing a cosmetic composition according to the exemplary embodiment mentioned above includes the method for producing microcapsules 10 and the like mentioned above. Thus, cosmetic compositions can be obtained which contain desired microcapsules 10 and the like.

While the microcapsule and method for producing the microcapsule, as well as the cosmetic composition and method for producing the cosmetic composition according to the present disclosure have been described above with reference to the exemplary embodiment and the examples, the present disclosure is not to be considered limited to the exemplary embodiment and the examples. Without departing from the spirit of the present disclosure, a variety of modifications conceived by those skilled in the art and applied to the exemplary embodiment and the examples, and another embodiment constructed by combining some constituents in the exemplary embodiment and the examples, are also included within the scope of the present disclosure.

The microcapsule and method for producing the microcapsule, as well as the cosmetic composition and method for producing the cosmetic composition according to the present disclosure can be applied to, for example, cosmetic compositions for use in hair care devices such as a drier, a hair iron, and a steamer.

## Claims

1. A microcapsule comprising a core substance and a wall substance including the core substance in the wall substance,
wherein
the core substance is an anionic hydrophobic substance,
the wall substance is a cationic polymer, and
the microcapsule is smaller than 200 µm in particle size.

2. The microcapsule according to claim 1, wherein the core substance is liquid.

3. The microcapsule according to claim 1 or 2, wherein the core substance contains a fatty acid having a carbon number of 5 or more at 80% or more in percent by weight.

4. The microcapsule according to any one of claims 1 to 3, wherein the wall substance is cross-linked.

5. The microcapsule according to any one of claims 1 to 4, wherein the wall substance includes a plurality of layers.

6. A cosmetic composition comprising a plurality of the microcapsules according to any one of claims 1 to 5 and a water phase that disperses the plurality of microcapsules,
wherein the plurality of microcapsules have an average particle size of 3 µm ± 1 µm and a standard deviation of 0.3 ± 0.1.

7. The cosmetic composition according to claim 6, wherein the water phase has a conductivity of higher than 7.5 µS/cm and lower than 1000 µS/cm.

8. The cosmetic composition according to claim 6 or 7, wherein the microcapsules are equal to the water phase in specific gravity.

9. A method for producing a microcapsule comprising a core substance and a wall substance including the core substance in the wall substance, the method comprising:
an oil-in-water emulsion solution preparation step of preparing an oil-in-water emulsion solution of the core substance which is an anionic hydrophobic substance by dispersing, in a water phase, the core substance; and
an inclusion step of including, in the wall substance which is a cationic polymer, microparticles of the core substance in the oil-in-water emulsion solution by mixing the oil-in-water emulsion solution with the wall substance.
wherein the microcapsule is smaller than 200 µm in particle size.

10. The method for producing a microcapsule according to claim 9, wherein the oil-in-water emulsion solution preparation step further includes a fatty acid addition step of adding, to the core substance, a fatty acid having a carbon number of 5 or more.

11. The method for producing a microcapsule according to claim 10, the method further comprising a cross-linking step of making the wall substance cross-linked.

12. The method for producing a microcapsule according to any one of claims 9 to 11, the method further comprising, after the inclusion step, a dehydration step of dehydrating the wall substance of the microcapsule with a poor solvent.

13. A method for producing a cosmetic composition, the method comprising the method for producing a microcapsule according to any one of claims 9 to 12.
